# EUROPEAN PATENT SPECIFICATION

(11) **EP 0 819 131 B1**
(45) Date of publication and mention of the grant of the patent: **12.03.2003**
(21) Application number: 96911996.5
(22) Date of filing: 02.04.1996
(51) Int. Cl.: C07D 501/12

(54) **PURIFICATION PROCESS**
REINIGUNGSVERFAHREN
PROCEDE DE PURIFICATION

(30) Priority: 03.04.1995 AT 59495
(43) Date of publication of application: 21.01.1998
(73) Proprietor: BIOCHEMIE GESELLSCHAFT M.B.H., 6250 Kundl (AT)
(72) Inventor: MILLER, Ludwig, A-6020 Innsbruck (AT); WIESER, Josef, A-6330 Kufstein (AT)
(74) Representative: Wymann, Gérard
(86) International application number: EP9601449
(87) International publication number: WO96031516

(56) References cited:
- CHEMICAL ABSTRACTS, vol. 104, no. 26, 30 June 1986 Columbus, Ohio, US; abstract no. 229890d, page 318; column r; XP002008278 & DIANDU YU HUANBAO, vol. 6, no. 1, 1986, pages 21-25,
- CHEMICAL ABSTRACTS, vol. 93, no. 20, 17 November 1980 Columbus, Ohio, US; abstract no. 191570p, page 329; column l; XP002008279 & JP,A,08 051 486 (NITTETSU KAKOKI CO, LTD) 15 April 1980

## Description

This invention relates to a purification process, in particular to a process for the removal of a fluoride from spent liquors obtained in a process for the production of a cephalosporin in which boron trifluoride is used.

Boron trifluoride may be used, *e.g.* as Lewis acid, in substitution reactions of organic compounds, for example in the production of cephalosporins. For example, the substitution of the 3-acetoxy group in 7-aminocephalosporanic acid (7-ACA) by the residue of an S- or O-nucleophile is an important reaction in a process for the production of cephalosporin antibiotics. If such a reaction is effected in water-free solvents and boron trifluoride is used as Lewis acid the reaction may result in high yields and purities of the corresponding product, *e.g.* of 7-ACA substituted by the residue of a nucleophile, for example in position 3.
In such a substitution reaction, for example between 7-ACA and a nucleophile, large amounts of fluoride which is meant to include boron-trifluoride or boron-trifluoride which is already partially hydrolysed, for example BF₂OH, BF(OH)₂ as well as free fluoride and tetrafluoroborate, containing spent liquor has to be disposed of after isolation of the substituted 7-ACA reaction product, since 7-ACA and boron trifluoride are to be employed at least in equivalent amounts. Thus, in the reaction of 7-ACA with thioles according to GB 1 565 941, 2 to 7 mols of boron trifluoride are used per mol of 7-ACA, and in the reaction of 7-ACA with methanol according to EP-0 204 657, more than 8 mols of boron trifluoride are used per mol of 7-ACA.

A conventional method of removing fluoride (in the meaning stated above) from spent liquors is the conversion thereof into calcium fluoride which is rather insoluble in water by total hydrolysis in the presence of a hydrate of CaO. It was found that this method may indeed also be carried out with a spent liquor obtained after reaction of a cephalosporin in the presence of boron trifluoride and isolation of the cephalosporin product, but this method has several disadvantages. First of all it was found that a spent liquor has to be heated at the boiling point of the spent liquor for a long time, *e.g.* up to about 7 hours. In addition, hydrolysis must be carried out in an acidic pH range, and therefore, owing to the long duration of hydrolysis and the high temperatures, corrosion damage of the reaction apparatus cannot be avoided. Secondly, it has been found that the calcium fluoride method yields a poorly filterable product with a content of about 50% of CaF₂ only, since other poorly-soluble calcium salts, *e.g.* from organic decomposition products, are formed. This product thus cannot be re-used. Therefore, a large amount of calcium fluoride waste has to be disposed.

It has now surprisingly been found a new, ecological, economical and simple process for the recovery of fluoride from a spent liquor, *e.g.* in the preparation of a cephalosporin in which fluoride is practically quantitatively converted into one single defined compound which may be precipitated as a poorly-soluble salt at low temperatures and in short time. This salt may be obtained in such good quality that it can be re-used, *e.g.* for the production of boron trifluoride. Thus, boron trifluoride used to produce, for example, 3-substituted 7-aminocephalosporanic acid derivatives may be regenerated by means of this process. Apart from the economical aspect, the ecological advantage is enormous. Additionally, free fluoride may also practically quantitatively be removed.

The present invention provides therefore in one aspect a process for the removal of a fluoride from a spent liquor obtained in a process for the production of a cephalosporin in which boron trifluoride is used, characterized in that fluoride is converted into a salt of tetrafluoroborate by adding a salt-forming agent and the salt of tetrafluoroborate is precipitated in the spent liquor.

Fluoride includes boron-trifluoride or boron-trifluoride which is already partially hydrolysed, for example BF₂OH, BF(OH)₂, as well as free fluoride and tetrafluoroborate.
A spent liquor may be the reaction mixture after isolation of the organic compound, *e.g.* a mother liquor.
A process for the production of acephalosporin in which boron trifluoride is used may be, for example, a process wherein boron trifluoride is used, *e.g.* as a catalyst (Lewis acid), for example, in a nucleophilic substitution reaction.

In a further aspect of the present invention the process for the production of a cephalosporin is one in which boron trifluoride is used in a nucleophilic substitution reaction.

Particularly, the cephalosporin is for example 7-aminocephalosporanic acid or a derivative thereof. A derivative of 7-ACA includes, for example, 7-ACA which may be N-substituted in position 7 by groups which are inert under the reaction conditions in the production of the cephalosporin in which boron trifluoride is used, for example by acyl groups, and the carboxylic group in position 4 of 7-ACA may be, independently of the substituent in position 7, in free form, salt form or protected, *e.g.* esterified. In particular the cephalosporin may be 7-aminocephalosporanic acid.

A process for the production of a cephalosporin in which boron trifluoride is used may be, for example, a nucleophilic substitution reaction at the methyl group in position 3 of 7-aminocephalosporanic acid or derivative thereof.

The spent liquor obtained after isolation of the corresponding cephalosporin may contain one single or several organic solvents and water. A reaction of an organic compound in the presence of boron trifluoride is normally carried out under practically water-free conditions. The spent liquor may thus be substantially water-free. Suitable solvents or solvent mixtures are known, for example from GB 1 565 941 or EP-0 204 657. Water may additionally be used in the isolation of the cephalosporin obtained after reaction; the spent liquor may thus contain also water. The process according to this invention is independent on the solvent system used. Parts of the solvent system may be distilled off, if desired.

The process according to the present invention may be carried out as follows:
The spent liquor obtained after isolation of the cephalosporinafter reaction may optionally be pre-treated, *i.e.* treatment prior to the isolation of the salt of tetrafluoroborate, to improve the purity of the salt of boron tetrafluoroborate to be obtained. This may be carried out in conventional manner. Pre-treatment may include, for example, filtration of the spent liquor, *e.g.* over active carbon, for example trough an activated carbon filter, *e.g.* to remove insoluble impurities and/or to decolorize the spent liquor. This may be done before conversion of the fluoride into a salt of tetrafluoroborate. Pre-treatment includes further treatment with hydrogen peroxide, for example at a weakly acidic or alkaline pH, or, removal of part of the solvent (system) from the spent liquor, for example by distillation; this may be done before, after, or during conversion of the fluoride into a salt of tetrafluoroborate.

In another aspect of the present invention the spent liquor is pre-treated before isolation of the salt of tetrafluoroborate, for example
a. with hydrogen peroxide, and/or
b. by filtration through an activated carbon filter, and/or
c. by distilling off at least a part of the spent liquor.

Particularly, a tetrafluoroborate may be precipitated in the spent liquor. For the conversion of fluoride into a salt, a salt forming agent, for example an inorganic salt, *e.g.* an alkali salt or an ammonium salt, or, for example, ammonium under acidic conditions, preferably a potassium salt may be used. A potassium salt includes *e.g.* potassium chloride, potassium hydroxide, potassium sulphate, caustic potash solution or potassium fluoride, for example potassium chloride, caustic potash solution, potassium hydroxide. Or, for example potassium chloride, potassium sulphate, caustic potash solution or potassium fluoride at a pH value of between 0.5 and 3, preferably between 1 and 2. The optimal salt forming agent may be chosen case by case dependent on the composition of the spent liquor.
Precipitation may be effected using caustic potash solution without the addition of an acid during addition of the salt. The liquor may be acidified before isolation of the potassium tetrafluoroborate. In this case, ca. 70% of the fluoride present in the spent liquor is obtained as the potassium tetrafluoroborate, but more fluoride is present in the filtrate, and this can be precipitated afterwards as calcium fluoride. The salt forming agent may be used in equivalent amounts in respect to the amount of fluoride present in the spent liquor or an excess may be used. The salt forming agent may be added to the spent liquor in solid form or in solution.

In another aspect of the present invention the salt of tetrafluoroborate is preferably potassium tetrafluoroborate.

The addition temperature of the salt forming agent may be dependent on further parts of the spent liquor which may be recovered, for example by parallel distillation beside salt formation of the tetrofluoroborate. Addition may be at temperatures below, for example between about 0 to 20°C, for example 10°C, at, or over room temperature, for example at an appropriate temperature in the distillation residue from which a part of the spent liquor may be distilled off, *e.g.* up to about 100° C, for example 90° C. In a particular aspect of the present invention the salt forming agent may be added at room temperature, *i.e.* without cooling or heating.
In most cases an acidic pH of the spent liquor may be an advantage. The pH may be in the range, *e.g*. from about 0.5 to 3, particularly from 1 to 2. The spent liquor may optionally be acidified, in particular using an inorganic acid, such as hydrochloric acid, sulphuric acid, preferably sulphuric acid before or after pre-treatment and before or after salt addition.

In another aspect of the invention the liquor is acidified.

The tetrafluoroborate salt may precipitate and can be filtered off very easily. It may be isolated in a yield of up to 99% in highly pure form, *e.g*. in a purity of 99%, for example in crystalline form.

By addition of potassium fluoride or of an equivalent amount of hydrogen fluoride, optionally remaining boric acid which may be present in the spent liquor due to hydrolysis effects may also be converted into the tetrafluoroborate anion, so that boron can also be removed from the spent liquor (beside, for example after fluoride) practically completely.

In another aspect the present invention provides a process for the production of tetrafluoroborate wherein fluoride is removed from a spent liquor and, if desired, remaining boron present in the liquor is removed by addition of potassium fluoride or hydrogen fluoride.

In a particular process of the invention the spent liquor obtained after nucleophilic substitution of the 3-acetoxy group in position 3 of 7-ACA in which boron trifluoride is used is pre-treated by filtration over active carbon before addition of the salt forming agent and/or a part of the solvent (system) is distilled off and/or hydrogen peroxide is added. Potassium chloride, potassium hydroxide or caustic potash solution is added at temperatures from room temperature up to about 90°C in the distillation residue, *i.e.* in the case that a part of the spent liquor is distilled off during formation of the potassium tetrafluoroborate. The mixture is optionally cooled to temperatures from -10° to about 15 °C, preferably from 0 to 10°C. The spent liquor may optionally be acidified, in particular using an inorganic acid, such as hydrochloric acid, sulphuric acid at an appropriate stage.

Potassium tetrafluoroborate is obtained in crystalline form, for example in form of white crystalls. The quality of the potassium tetrafluoroborate obtained is such excellent that it may be re-used without further purification, for example for the preparation of further boron trifluoride, usable, for example in a further reaction of an organic compound in which boron trifluoride is desired. Conversation of potassium tetrafluoroborate into boron trifluoride may be carried out according to conventional methods, for example by thermic treatment.

In a further aspect the present invention provides a process as described elsewhere above wherein the tetrafluoroborate is used to produce boron trifluoride and a process for the generation of boron trifluoride from a tetrafluoroborate characterized in that the tetrafluoroborate is produced according to a process as described elsewhere above.

In a further aspect the present invention provides a process for the removal of a fluoride from spent liquors obtained in processes in which boron trifluoride is used as a reagent in the production of 3-substituted 7-aminocephalosporanic acid derivatives, characterized in that the fluoride is precipitated as potassium tetrafluoroborate by adding a salt-forming agent.
In the following examples, which illustrate the invention more fully without limiting its scope, all temperatures are given in degrees celsius.

### Example 1:

1 litre of mother liquor, which is obtained analogously to the first example of GB 1 565 941 by reacting 7-ACA in sulpholane with 5-mercapto-1-methyltetrazole and boron trifluoride, and which contains ca. 4% fluoride in the meaning as stated above, is adjusted to pH 1.0 with concentrated hydrochloric acid and filtered through a frit covered with 2 g of filter aid and 10 g of activated carbon. The filtrate is mixed whilst stirring and cooling at 20 to 25° with 250 ml of a 20% aqueous potassium chloride solution. The pH is kept at 1.2 to 1.6 by simultaneously adding concentrated hydrochloric acid. The precipitating suspension obtained is stirred for 2 hours at room temperature, then cooled to 5° and after 30 minutes is filtered. The thus isolated potassium tetrafluoroborate is washed with little cold water and dried in a vacuum at 50°. 95% of the fluoride present in the mother liquor are obtained in pure crystalline form as the potassium tetrafluoroborate.

### Example 2:

1 litre of mother liquor, which is obtained as described in the example of EP-0 204 657 by reacting 7-ACA in sulpholane with methanol and boron trifluoride, and which contains ca. 10% fluoride in the meaning as stated above, is distilled at normal pressure in a distillation apparatus until the methanol present in the mother liquor is distilled off. At a temperature of the distillation residue of 70°, 240 g of 45% caustic potash solution is continuously added through a dropping funnel over the course of ca. 20 minutes, and distillation continues until the distillation residue reaches a temperatures of 90°. The triethylamine used as the base in the process of EP-0 204 657 is obtained as a distillate, and at the same time the potassium tetrafluoroborate precipitates in the distillation residue. 300 g of 37% hydrogen peroxide are added to the distillation residue which is at a temperature of 80°. Within 10 minutes the reaction mixture which is at first dark brown is lightened to a yellow-orange suspension. This suspension is adjusted to pH 1.5 with ca. 80 g of concentrated sulphuric acid and stirred for 60 minutes at 0°. The precipitated potassium tetrafluoroborate is filtered off, washed with a little water and dried. 99.3% of the fluoride present in the mother liquor are obtained as the potassium tetrafluoroborate in the form of a white, crystalline powder.

### Example 3:

To 1 litre of mother liquor, which is obtained analogously to the first example of GB 1 565 941 by reacting 7-ACA in acetonitrile with 2-methyl-3-mercapto-triazine-5,6-dione and boron trifluoride, and which contains ca. 4% fluoride in the meaning as stated above, are added under stirring 40 ml of 45% caustic potash solution. 10 ml of 70% hydrogen peroxide are added slowly over the course of 10 minutes. The reaction mixture is stirred for 1 hour at room temperature, adjusted to pH 6.5 - 6.8 with 76% sulphuric acid, and the acetonitrile is distilled off at normal pressure. The orange-yellow residue of distillation is cooled to room temperature and adjusted to pH 1.5 with 76% sulphuric acid. After stirring for two hours at 10°, the potassium tetrafluoroborate is filtered off, washed with a little cold water and dried in a drying chamber at 50°. 96% of the fluoride present in the mother liquor are obtained as the potassium tetrafluoroborate in the form of a coarsely-crystalline, pure white powder.

## Claims

1. A process for the removal of a fluoride from spent liquors obtained in a process for the production of a cephalosporin in which boron trifluoride is used, **characterized in that** fluoride is converted into a salt of tetrafluoroborate by adding a salt-forming agent and the salt of tetrafluoroborate is precipitated in the spent liquor.

2. A process for the removal of a fluoride from spent liquors obtained in a process for the production of a cephalosporin according to claim 1, in which the cephalosporin is a 7-aminocephalosporanic acid.

3. A process according to any one of claims 1 to 2, **characterized in that** the salt of tetrafluoroborate is potassium tetrafluoroborate.

4. A process according to any one of claims 1 to 3, **characterized in that** the spent liquor is acidified before isolation of the salt of tetrafluoroborate.

5. A process according to anyone of claims 1 to 4, **characterized in that** the spent liquor is pre-treated before isolation of the salt of tetrafluoroborate.

6. A process according to claim 5 wherein the liquor is pre-treated
a. with hydrogen peroxide; and/or
b. by filtration through an activated carbon filter, and/or
c. by distilling off at least a part of the spent liquor.

7. A process according to any one of claims 1 to 6, wherein remaining boron present in the spent liquor is removed by addition of potassium fluoride or hydrogen fluoride.

8. A process according to any one of claims 1 to 7 which is further **characterized in that** tetrafluoroborate is used to produce boron trifluoride.

9. A process for the removal of a fluoride from spent liquors obtained in processes in which boron trifluoride is used as a reagent in the production of 3-substituted 7-aminocephalosporanic acid derivatives, **characterized in that** the fluoride is precipitated as potassium tetrafluoroborate by adding a salt-forming agent.

10. A process according to claim 9, **characterised in that** the potassium tetrafluoroborate precipitation is carried out using potassium salts such as potassium chloride, potassium sulphate, caustic potash solution or potassium fluoride at a pH value of between 1 and 2.

## Patentansprüche

1. Verfahren zur Entfernung eines Fluorids aus Abfallflüssigkeiten, die in einem Verfahren zur Herstellung eines Cephalosporins erhalten wurden, worin Bortrifluorid verwendet wird, **gekennzeichnet durch** Umwandlung des Fluorids in ein Tetrafluorboratsalz **durch** die Zugabe eines salzbildenden Mittels und Fällung des Tetrafluorboratsalzes in der Abfallflüssigkeit.

2. Verfahren zur Entfernung eines Fluorids aus Abfallflüssigkeiten, die in einem Verfahren zur Herstellung eines Cephalosporins nach Anspruch 1 erhalten wurde, worin das Cephalosporin eine 7-Aminocephalosporansäure ist.

3. Verfahren nach einem der Ansprüche 1 bis 2, **dadurch gekennzeichnet, daß** das Tetrafluorboratsalz Kaliumtetrafluorborat ist.

4. Verfahren nach einem der Ansprüche 1 bis 3, **dadurch gekennzeichnet, daß** die Abfallflüssigkeit vor der Isolierung des Tetrafluorboratsalzes angesäuert wird.

5. Verfahren nach einem der Ansprüche 1 bis 4, **dadurch gekennzeichnet, daß** die Abfallflüssigkeit vor der Isolierung des Tetrafluorboratsalzes vorbehandelt wird.

6. Verfahren nach Anspruch 5, worin die Flüssigkeit vorbehandelt wird
a. mit Wasserstoffperoxid und/oder
b. durch Filtration durch ein Aktivkohlefilter, und/oder
c. durch Abdestillation zumindest eines Teils der Abfallflüssigkeit.

7. Verfahren nach einem der Ansprüche 1 bis 6, worin das verbleibende Bor, das in der Abfallflüssigkeit vorhanden ist, durch die Zugabe von Kaliumfluorid oder Fluorwasserstoff entfernt wird.

8. Verfahren nach einem der Ansprüche 1 bis 7, das ferner **dadurch gekennzeichnet ist, daß** Tetrafluorborat zur Bildung von Bortrifluorid verwendet wird.

9. Verfahren zur Entfernung eines Fluorids aus Abfallflüssigkeiten, die in Verfahren erhalten wurden, worin Bortrifluorid als Reagenz zur Herstellung von 3-substituierten 7-Aminocephalosporansäurederivaten verwendet wird, **dadurch gekennzeichnet, daß** das Fluorid als Kaliumtetrafluorborat durch die Zugabe eines salzbildenden Mittels gefällt wird.

10. Verfahren nach Anspruch 9, **dadurch gekennzeichnet, daß** die Kaliumtetrafluorboratfällung mittels Kaliumsalzen, wie Kaliumchlorid, Kaliumsulfat, Ätzkalilösung oder Kaliumfluorid bei einem pH Wert zwischen 1 und 2 ausgeführt wird.

## Revendications

1. Procédé d'élimination d'un fluorure dans des liqueurs usées obtenues dans un procédé de production d'une céphalosporine dans lequel on utilise du trifluorure de bore, **caractérisé en ce que** le fluorure est converti en un sel de tétrafluoroborate par addition d'un agent de formation de sel, et le sel de tétrafluoroborate est précipité dans la liqueur usée.

2. Procédé d'élimination d'un fluorure de liqueurs usées obtenues dans un procédé de production d'une céphalosporine suivant la revendication 1, dans lequel la céphalosporine est un acide 7-aminocéphalosporanique.

3. Procédé suivant l'une quelconque des revendications 1 et 2, **caractérisé en ce que** le sel de tétrafluoroborate est le tétrafluoroborate de potassium.

4. Procédé suivant l'une quelconque des revendications 1 à 3, **caractérisé en ce que** la liqueur usée est acidifiée avant l'isolement du sel de tétrafluoroborate.

5. Procédé suivant l'une quelconque des revendications 1 à 4, **caractérisé en ce que** la liqueur usée est prétraitée avant l'isolement du sel de tétrafluoroborate.

6. Procédé suivant la revendication 5, dans lequel la liqueur est prétraitée
a. avec du peroxyde d'hydrogène ; et/ou
b. par filtration à travers un filtre de charbon activé, et/ou
c. en éliminant par distillation au moins une partie de la liqueur usée.

7. Procédé suivant l'une quelconque des revendications 1 à 6, dans lequel le bore résiduel présent dans la liqueur usée est éliminé par addition de fluorure de potassium ou de fluorure d'hydrogène.

8. Procédé suivant l'une quelconque des revendications 1 à 7, qui est en outre **caractérisé en ce que** le tétrafluoroborate est utilisé pour produire du trifluorure de bore.

9. Procédé d'élimination d'un fluorure dans des liqueurs usées obtenues dans des procédés dans lesquels on utilise du trifluorure de bore comme réactif dans la production de dérivés d'acide 7-aminocéphalosporanique 3-substitués, **caractérisé en ce que** le fluorure est précipité sous forme de tétrafluoroborate de potassium paraddition d'un agent de formation de sel.

10. Procédé suivant la revendication 9, **caractérisé en ce que** la précipitation du tétrafluoroborate de potassium est réalisée en utilisant des sels de potassium tels que le chlorure de potassium, le sulfate de potassium, une solution de potasse caustique ou le fluorure de potassium à une valeur de pH comprise entre 1 et 2.
